# EUROPEAN PATENT APPLICATION

(11) **EP 2 363 149 A1**
(43) Date of publication of application: **07.09.2011**
(21) Application number: 10179650.6
(22) Date of filing: 02.05.2005
(51) Int. Cl.: A61K 45/06, A61P 37/06

(54) **Combinations comprising a S1P receptor agonist and a JAK3 kinase inhibitor**

(30) Priority: 03.05.2004 US 567677 P; 21.07.2004 US 590061 P
(62) Divisional of application: 05741179.5
(71) Applicant: Novartis AG, 4056 Basel (CH); Novartis Pharma GmbH, 1230 Wien (AT)
(72) Inventor: LAKE, Philip, Morris Plains, NJ 07950 (US)
(74) Representative: Rouquayrol, Céline Hélène

(57) **Abstract**

The invention provides a pharmaceutical combination comprising:
a) at least one S1P receptor agonist, and
b) at least one JAK3 kinase inhibitor
and a method for treating or preventing autoimmune diseases or cell, tissue or organ graft rejection using such a combination.

## Description

### Combinations

The present invention relates to a pharmaceutical combination comprising at least one sphingosine-1-phosphate (S1P) receptor agonist and at least one JAK3 kinase inhibitor and the uses of such a combination e.g. in autoimmune diseases, e.g. in preventing or treating type I diabetes mellitus and disorders associated therewith, or in transplantation.

In spite of numerous treatment options for organ transplant and autoimmune disease patients, there remains a need for effective and safe immunosuppressive agents and a need for their preferential use in combination therapy.

It has now been found that a combination comprising at least one S1P receptor agonist or modulator and a Janus Kinase 3 (JAK3) kinase inhibitor, e.g. as defined below, has a beneficial effect on autoimmune diseases, e.g. type I diabetes and the disorders associated therewith, or graft rejection.

S1P receptor agonists or modulators are compounds which signal as agonists at one or more sphingosine-1 phosphate receptors, e.g. S1P1 to S1P8. Agonist binding to a S1P receptor may e.g. result in dissociation of intracellular heterotrimeric G-proteins into Gα-GTP and Gβγ-P, and/or increased phosphorylation of the agonist-occupied receptor and activation of downstream signaling pathways/kinases.

The binding affinity of S1P receptor agonists or modulators to individual human S1P receptors may be determined in following assay:
S1P receptor agonist or modulator activities of compounds are tested on the human S1P receptors S1P₁, S1P₃, S1P₂, S1P₄ and S1P₅. Functional receptor activation is assessed by quantifying compound induced GTP [γ-³⁵S] binding to membrane protein prepared from transfected CHO or RH7777 cells stably expressing the appropriate human S1 P receptor.

The assay technology used is SPA (scintillation proximity based assay). Briefly, DMSO dissolved compounds are serially diluted and added to SPA- bead (Amersham-Pharmacia) immobilised S1P receptor expressing membrane protein (10-20µg/well) in the presence of 50 mM Hepes, 100 mM NaCl, 10 mM MgCl₂, 10 µM GDP, 0.1% fat free BSA and 0.2 nM GTP [γ-³⁵S] (1200 Ci/mmol). After incubation in 96 well microtiterplates at RT for 120 min, unbound GTP [γ-³⁵S] is separated by a centrifugation step. Luminescence of SPA beads triggered by membrane bound GTP [γ-³⁵S] is quantified with a TOPcount plate reader (Packard). EC₅₀s are calculated using standard curve fitting software. In this assay, the S1 P receptor agonists preferably have a binding affinity to S1P receptor <50 nM.

Preferred S1P receptor agonists or modulators are e.g. compounds which in addition to their S1P binding properties also have accelerating lymphocyte homing properties, e.g. compounds which elicit a lymphopenia resulting from a re-distribution, preferably reversible, of lymphocytes from circulation to secondary lymphatic tissue, without evoking a generalized immunosuppression. Naïve cells are sequestered; CD4 and CD8 T-cells and B-cells from the blood are stimulated to migrate into lymph nodes (LN) and Peyer's patches (PP).

The lymphocyte homing property may be measured in following Blood Lymphocyte Depletion assay:
A S1P receptor agonist or modulator or the vehicle is administered orally by gavage to rats. Tail blood for hematological monitoring is obtained on day -1 to give the baseline individual values, and at 2, 6, 24, 48 and 72 hours after application. In this assay, the S1P receptor agonist or modulator depletes peripheral blood lymphocytes, e.g. by 50%, when administered at a dose of e.g. < 20 mg/kg.

Examples of appropriate S1P receptor agonists or modulators are, for example:
- Compounds as disclosed in EP627406A1, e.g. a compound of formula I 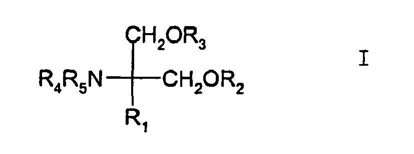 wherein R₁ is a straight- or branched (C₁₂₋₂₂)chain
- which may have in the chain a bond or a hetero atom selected from a double bond, a triple bond, O, S, NR₆, wherein R₆ is H, C₁₋₄alkyl, aryl-C₁₋₄alkyl, acyl or (C₁₋₄alkoxy)carbonyl, and carbonyl, and/or
   - which may have as a substituent C₁-₄alkoxy, C₂₋₄alkenyloxy, C₂₋₄alkynyloxy, arylC₁₋₄alkyl-oxy, acyl, C₁₋₄alkylamino, C₁₋₄alkylthio, acylamino, (C₁₋₄alkoxy)carbonyl, (C₁₋₄alkoxy)-carbonylamino, acyloxy, (C₁₋₄alkyl)carbamoyl, nitro, halogen, amino, hydroxyimino, hydroxy or carboxy; or
   R₁ is
- a phenylalkyl wherein alkyl is a straight- or branched (C₆₋₂₀)carbon chain; or
- a phenylalkyl wherein alkyl is a straight- or branched (C₁₋₃₀)carbon chain wherein said phenylalkyl is substituted by
- a straight- or branched (C₆₋₂₀)carbon chain optionally substituted by halogen,
- a straight- or branched (C₆₋₂₀)alkoxy chain optionally substitued by halogen,
- a straight- or branched (C₆₋₂₀)alkenyloxy,
- phenyl-C₁₋₁₄alkoxy, halophenyl-C₁₋₄alkoxy, phenyl-C₁₋₁₄alkoxy-C₁₋₁₄alkyl, phenoxy-C₁₋₄alkoxy or phenoxy-C₁₋₄alkyl,
- cycloalkylalkyl substituted by C₆₋₂₀alkyl,
- heteroarylalkyl substituted by C₆₋₂₀alkyl,
- heterocyclic C₆₋₂₀alkyl or
- heterocyclic alkyl substituted by C₂₋₂₀alkyl,
   and wherein
   the alkyl moiety may have
- in the carbon chain, a bond or a heteroatom selected from a double bond, a triple bond, O, S, sulfinyl, sulfonyl, or NR₆, wherein R₆ is as defined above, and
- as a substituent C₁₋₄alkoxy, C₂₋₄alkenyloxy, C₂₋₄alkynyloxy, arylC₁₋₄alkyloxy, acyl, C₁₋₄alkylamino, C₁₋₄alkylthio, acylamino, (C₁₋₄alkoxy)carbonyl, (C₁₋₄alkoxy)carbonylamino, acyloxy, (C₁₋₄alkyl)carbamoyl, nitro, halogen, amino, hydroxy or carboxy, and
   each of R₂, R₃, R₄ and R₅, independently, is H, C₁₋₄ alkyl or acyl
   or a pharmaceutically acceptable salt or hydrate thereof;
- Compounds as disclosed in EP 1002792A1, e.g. a compound of formula II 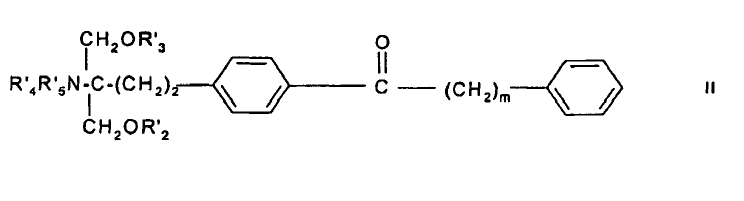 wherein m is 1 to 9 and each of R'₂, R'₃, R'₄ and R'₅, independently, is H, C₁₋₆alkyl or acyl, or a pharmaceutically acceptable salt or hydrate thereof;
- Compounds as disclosed in EP0778263 A1, e.g. a compound of formula III 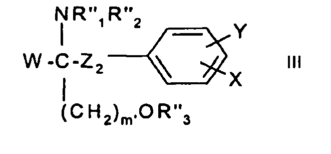 wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH;
   X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆alkyl, OH, C₁₋₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₋₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
   each of p and q, independently, is an integer of 1 to 20, with the proviso of 6≤p+q≤23, m' is 1, 2 or 3, n is 2 or 3,
   each of R"₁, R"₂, R"₃ and R"₄, independently, is H, C₁₋₄alkyl or acyl,
   or a pharmaceutically acceptable salt or hydrate thereof,
- Compounds as disclosed in WO02/18395, e.g. a compound of formula IVa or IVb 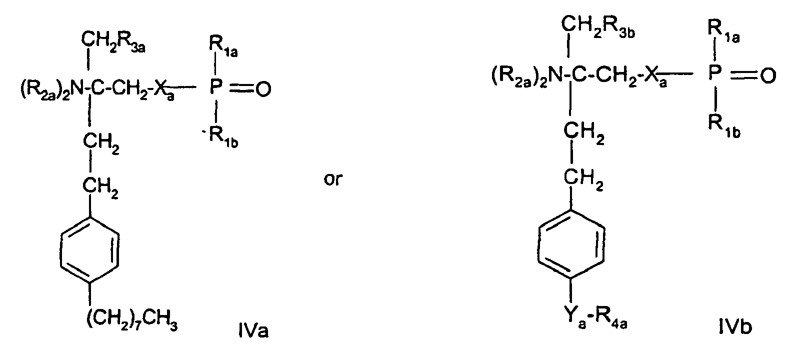 wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄₋₁₄)alkyl or (C₄₋₁₄)alkenyl; or a pharmaceutically acceptable salt or hydrate thereof;
- Compounds as disclosed in WO 02/076995, e.g. a compound of formula V 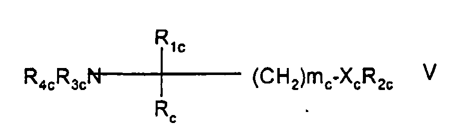 wherein
   - m_{c}: is 1, 2 or 3;
   - X_{c}: is O or a direct bond;
   - R_{1c}: is H; C₁₋₆alkyl optionally substituted by OH, acyl, halogen, C₃₋₁₀cycloalkyl, phenyl or hydroxy-phenylene; C₂₋₈alkenyl; C₂₋₆alkynyl; or phenyl optionally substituted by OH;
   - R_{2c}: is 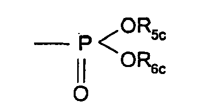 wherein R_{5c} is H or C₁₋₄alkyl optionally substituted by 1, 2 or 3 halogen atoms, and R_{6c} is H or C₁₋₄alkyl optionally substituted by halogen;
   each of R_{3c} and R_{4c}, independently, is H, C₁₋₄alkyl optionally substituted by halogen, or acyl, and
   - R_{c}: is C₁₃₋₂₀alkyl which may optionally have in the chain an oxygen atom and which may optionally be substituted by nitro, halogen, amino, hydroxy or carboxy; or a residue of formula (a) 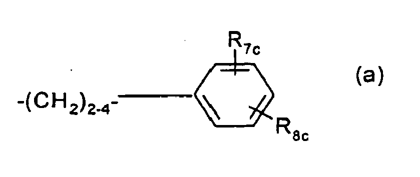 wherein R_{7c} is H, C₁₋₄alkyl or C₁₋₄alkoxy, and R_{8c} is substituted C₁₋₂₀alkanoyl, phenylC₁₋₁₄alkyl wherein the C₁₋₁₄alkyl is optionally substituted by halogen or OH, cycloalkylC₁₋₁₄alkoxy or phenylC₁₋₁₄alkoxy wherein the cycloalkyl or phenyl ring is optionally substituted by halogen, C₁₋₄alkyl and/or C₁₋₄alkoxy, phenylC₁₋₁₄alkoxy-C₁₋₁₄alkyl, phenoxyC₁₋₁₄alkoxy or phenoxyC₁₋₁₄alkyl,
   - R_{c}: being also a residue of formula (a) wherein R_{8c} is C₁₋₁₄alkoxy when R_{1c} is C₁₋₄alkyl, C₂₋₆alkenyl or C₂₋₆alkynyl,
   or a compound of formula VI 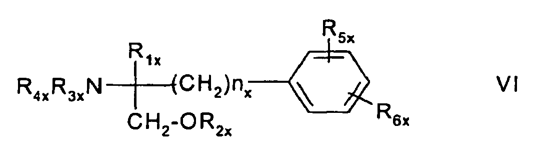 wherein
   - nₓ: is 2, 3 or 4
   - R₁ₓ: is H; C₁₋₆alkyl optionally substituted by OH, acyl, halogen, cycloalkyl, phenyl or hydroxy-phenylene; C₂₋₆alkenyl; C₂₋₆alkynyl; or phenyl optionally substituted by OH;
   - R₂ₓ: is H, C₁₋₄ alkyl or acyl
   - each of R₃ₓ: and R₄ₓ, independently is H, C₁₋₄alkyl optionally substituted by halogen or acyl,
   - R₅ₓ: is H, C₁₋₄alkyl or C₁₋₄alkoxy, and
   - R₆ₓ: is C₁₋₂₀ alkanoyl substituted by cycloalkyl; cyloalkylC₁₋₁₄alkoxy wherein the cycloalkyl ring is optionally substituted by halogen, C₁₋₄alkyl and/or C₁₋₄alkoxy; phenylC₁₋₁₄alkoxy wherein the phenyl ring is optionally substituted by halogen, C₁₋₄alkyl and/or C₁₋₄alkoxy,
   - R₆ₓ: being also C₄₋₁₄alkoxy when R₁ₓ is C₂₋₄alkyl substituted by OH, or pentyloxy or hexyloxy when R₁ₓ is C₁₋₄akyl,
   provided that R₆ₓ is other than phenyl-butylenoxy when either R₅ₓ is H or R₁ₓ is methyl,
   or a pharmaceutically acceptable salt or hydrate thereof;
- Compounds as disclosed in WO02/06268A1, e.g. a compound of formula VII 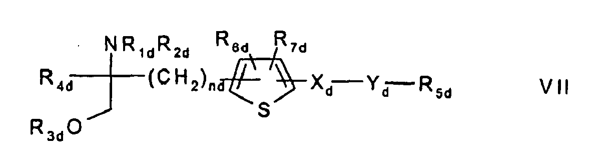
   wherein each of R_{1d} and R_{2d}, independently, is H or an amino-protecting group;
   R_{3d} is hydrogen, a hydroxy-protecting group or a residue of formula 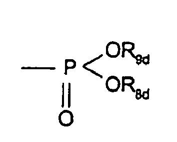
   R_{4d} is C₁₋₄alkyl;
   n_{d} is an integer of 1 to 6;
   X_{d} is ethylene, vinylene, ethynylene, a group having a formula - D-CH₂- (wherein D is carbonyl, - CH(OH)-, O, S or N), aryl or aryl substituted by up to three substitutents selected from group a as defined hereinafter;
   Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
   R_{5d} is hydrogen, C₃₋₆cycloalkyl, aryl, heterocyclic group, C₃₋₆cycloalkyl substituted by up to three substituents selected from groups a and b, aryl substituted by up to three substituents selected from groups a and b, or heterocyclic group substituted by up to three substituents selected from groups a and b;
   each of R_{6d} and R_{7d}, independently, is H or a substituent selected from group a;
   each of R_{8d} and R_{9d}, independently, is H or C₁₋₄alkyl optionally substituted by halogen;
   <group a > is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-C₁₋₄alkylamino, acylamino, cyano or nitro; and
   <group b > is C₃₋₆cycloalkyl, aryl or heterocyclic group, each being optionally substituted by up to three substituents selected from group a;
   with the proviso that when R_{5d} is hydrogen, Y_{d} is a either a single bond or linear C₁₋₁₀ alkylene, or a pharmacologically acceptable salt, ester or hydrate thereof;
- Compounds as disclosed in JP-14316985 (JP2002316985), e.g. a compound of formula VIII 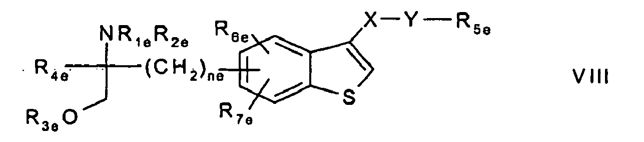
   wherein R₁ₑ,R₂ₑ,R₃ₑ,R₄ₑ,R₅ₑ,R₆ₑ,R₇ₑ, nₑ, Xₑ and Yₑ are as disclosed in JP-14316985; or a pharmacologically acceptable salt, ester or hydrate thereof;
- Compounds as disclosed in WO 03/29184 and WO 03/29205, e.g. compounds of formula IX 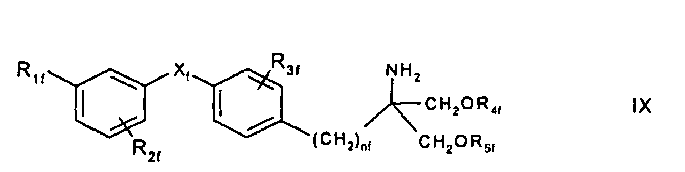
   wherein X₁ is O, S, SO or SO₂
   R_{1f} is halogen, trihalomethyl, OH, C₁₋₇alkyl, C₁₋₄alkoxy, trifluoromethoxy, phenoxy, cyclohexylmethyloxy, pyridylmethoxy, cinnamyloxy, naphthylmethoxy, phenoxymethyl, CH₂-OH, CH₂-CH₂-OH, C₁₋₄alkylthio, C₁₋₄alkylsulfinyl, C₁₋₄alkylsulfonyl, benzylthio, acetyl, nitro or cyano, or phenyl, phenylC₁₋₄alkyl or phenyl-C₁₋₄alkoxy each phenyl group thereof being optionally substituted by halogen, CF₃, C₁₋₄alkyl or C₁₋₄alkoxy;
   R₂, is H, halogen, trihalomethyl, C₁₋₄alkoxy, C₁₋₇alkyl, phenethyl or benzyloxy;
   R_{3f} H, halogen, CF₃, OH, C₁₋₇alkyl, C₁₋₄alkoxy, benzyloxy or C₁₋₄alkoxymethyl;
   each of R_{4f} and R_{5f}, independently is H or a residue of formula

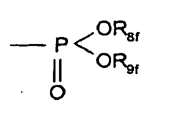

   wherein each of R_{8f} and R_{9f}, independently, is H or C₁₋₄alkyl optionally substituted by halogen;and
   n_{f} is an integer from 1 to 4;
   e.g. 2-amino-2-[4-(3-benzyloxyphenoxy)-2-chlorophenyl]propyl-1,3-propane-diol or 2-amino-2-[4-(benzyloxyphenylthio)-2- chlorophenyl]propyl-1,3-propane-diol, or a pharmacological salt or hydrate thereof;
- Compounds as disclosed in WO03/062252A1, e.g. a compound of formula X 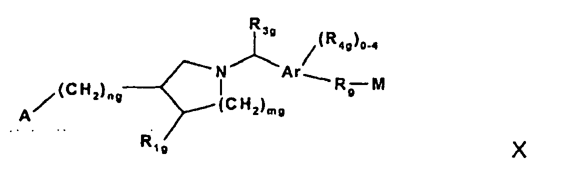 wherein
   Ar is phenyl or naphthyl; each of m₉ and n₉ independently is 0 or 1; A is selected from COOH, PO₃H₂, PO₂H, SO₃H, PO(C₁₋₃alkyl)OH and 1H-tetrazol-5-yl; each of R_{1g} and R_{2g} independently is H, halogen, OH, COOH or C₁₋₄alkyl optionally substituted by halogen; R_{3g} is H or C₁₋₄alkyl optionally substituted by halogen or OH; each R_{4g} independently is halogen, or optionally halogen substituted C₁₋₄alkyl or C₁₋₃alkoxy; and each of R_{g} and M has one of the significances as indicated for B and C, respectively, in WO03/062252A1;
- Compounds as disclosed in WO 03/062248A2, e.g. a compound of formula XI 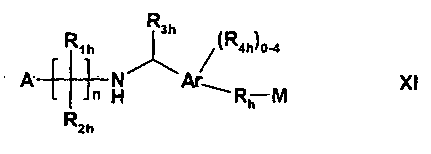
   wherein Ar is phenyl or naphthyl; n is 2,3 or 4; A is COOH, 1*H*-tetrazol-5-yl, PO₃H₂, PO₂H₂, - SO₃H or PO(R₅ₕ)OH wherein R₅ₕ is selected from C₁₋₄alkyl, hydroxyC₁₋₄alkyl, phenyl, -CO-C₁₋₃alkoxy and -CH(OH)-phenyl wherein said phenyl or phenyl moiety is opitonally substituted; each of R₁ₕ and R₂ₕ independently is H, halogen, OH, COOH, or optionally halogeno substituted C₁₋₆alkyl or phenyl; R₃ₕ is H or C₁₋₄alkyl optionally substituted by halogen and/ OH; each R₄ₕ independently is halogeno, OH, COOH, C₁₋₄alkyl, S(O)_{0,1 or 2}C₁₋₃alkyl, C₁-₃alkoxy, C₃₋₆cycloalkoxy, aryl or aralkoxy, wherein the alkyl portions may optionally be substituted by 1-3 halogens; and each of Rₕ and M has one of the significances as indicated for B and C, respectively, in WO03/062248A2.

According to a further embodiment of the invention, a S1P receptor agonist or modulator for use in a combination of the invention may also be a selective S1 P1 receptor, e.g. a compound which possesses a selectivity for the S1 P1 receptor over the S1 P3 receptor of at least 20 fold, e.g. 100, 500, 1000 or 2000 fold, as measured by the ratio of EC₅₀ for the S1P1 receptor to the EC₅₀ for the S1P3 receptor as evaluated in a ³⁵S-GTPγS binding assay, said compound having an EC₅₀ for binding to the S1P1 receptor of 100 nM or less as evaluated by the ³⁵S-GTPγS binding assay. Representative S1 P1 receptor agonists or modulators are e.g. the compounds listed in WO 03/061567, the contents of which being incorporated herein by reference, for instance a compound of formula

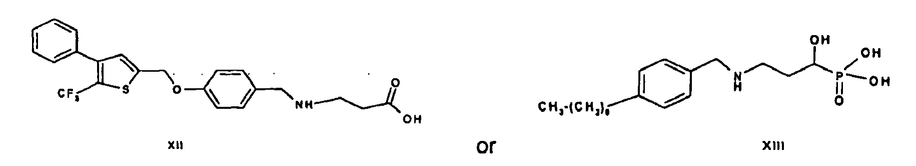

When the compounds of formulae I to XIII have one or more asymmetric centers in the molecule, the present invention is to be understood as embracing the various optical isomers, as well as racemates, diastereoisomers and mixtures thereof are embraced. Compounds of formula III or IVb, when the carbon atom bearing the amino group is asymmetric, have preferably the R-configuration at this carbon atom.

The compounds of formulae I to XIII may exist in free or salt form. Examples of pharmaceutically acceptable salts of the compounds of the formulae I to XIII include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts of the combination of the present invention encompass hydrate and solvate forms.

Acyl as indicated above may be a residue R_{y}-CO- wherein Ry is C₁₋₈alkyl, C₃₋₆cycloalkyl, phenyl or phenyl-C₁₋₄alkyl. Unless otherwise stated, alkyl, alkoxy, alkenyl or alkynyl may be straight or branched.

Aryl may be phenyl or naphthyl, preferably phenyl.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

In the above formula of VII "heterocyclic group" represents a 5- to 7 membered heterocyclic group having 1 to 3 heteroatoms selected from S, O and N. Examples of such heterocyclic groups include the heteroaryl groups indicated above, and heterocyclic compounds corresponding to partially or completely hydrogenated heteroaryl groups, e.g. furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl, pyrazinyl, tetrahydropyranyl, morpholinyl, thiomorpholinyl, pyrrolidinyl, pyrrolyl, imidazolidinyl, pyrazolidinyl, piperidinyl, piperazinyl, oxazolidinyl, isoxazolidinyl, thiazolidinyl or pyrazolidinyl. Preferred heterocyclic groups are 5-or 6-membered heteroaryl groups and the most preferred heteocyclic group is a morpholinyl, thiomorpholinyl or piperidinyl group.

A preferred compound of formula I is 2-amino-2-tetradecyl-1,3-propanediol. A particularly preferred S1P receptor agonist of formula I is FTY720, i.e. 2-amino-2-[2-(4-octylphenyl) ethyl]propane-1,3-diol in free form or in a pharmaceutically acceptable salt form (referred to hereinafter as Compound A), e.g. the hydrochloride, as shown:

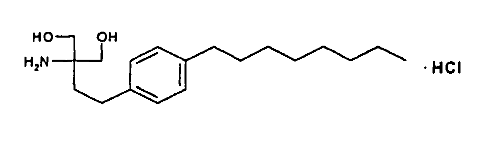

A preferred compound of formula II is the one wherein each of R'₂ to R'₅ is H and m is 4, i.e. 2-amino-2-{2-[4-(1-oxo-5-phenylpentyl)phenyl]ethyl}propane-1,3-diol, in free form or in pharmaceutically acceptable salt form (referred to hereinafter as Compound B), e.g the hydrochloride.

A preferred compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound C), e.g. the hydrochloride. The R-enantiomer is particularly preferred.

A preferred compound of formula IVa is the FTY720-phosphate (R₂ₐ is H, R₃ₐ is OH, Xₐ is O, R₁ₐ and R_{1b} are OH). A preferred compound of formula lVb is the Compound C-phosphate (R₂ₐ is H, R_{3b} is OH, Xₐ is O, R₁ₐ and R_{1b} are OH, Yₐ is O and R₄ₐ is heptyl). A preferred compound of formula V is Compound B-phosphate.

A preferred compound of formula V is phosphoric acid mono-[(R)-2-amino-2-methyl-4-(4-pentyloxy-phenyl)-butyl]ester.

A preferred compound of formula VIII is (2R)-2-amino-4-[3-(4-cyclohexyloxybutyl)-benzo[b]thien-6-yl]-2-methylbutan-1-ol.

JAK3 is an enzyme which is primarily expressed in T and B cells and plays a critical role in T cell development and function. JAK3 kinase inhibitors are e.g. compounds having an IC₅₀ value < 5 µM, preferably < 1 µM, more preferably < 0.1 µM in the following assays:

### Interleukin-2 (IL-2) dependent proliferation assays with CTL/L and HT-2 cells

The IL-2 dependent mouse T cell lines CTUL and HT-2 are cultured in RPMI 1640 (Gibco 52400-025) supplemented with 10% Fetal Clone I (HyClone), 50µM 2-mercaptoethanol (31350-010), 50µg/ml gentamycine (Gibco 15750-037), 1 mM sodium pyruvate (Gibco 11360-039), non-essential amino acids (Gibco 11140-035; 100x) and 250U/ml mouse IL-2 (supernatant of X63-Ag8 transfected cells containing 50'000 U/ml mouse IL-2 according to Genzyme standard). Cultures are split twice a week 1:40.

Before use the cells are washed twice with culture medium without mouse IL-2. The proliferation assay is performed with 4000 CTUL cells/well or 2500 HT-2 cells/well in flat-bottom 96-well tissue culture plates containing appropriate dilutions of test compounds in culture medium with 50U/ml mouse IL-2. CTUL cultures are incubated at 37°C for 24 h and HT-2 cultures are incubated for 48 h. After addition of 1 µCi ³H-thymidine and a further overnight incubation cells are harvested onto fibre filters and radioactivity is counted.

### Interleukin-2 dependent proliferation of human peripheral blood mononuclear cells

Human peripheral blood mononuclear cells are isolated on Ficoll from buffy coats with unknown HLA type (Blutspendezentrum, Kantonsspital, Basel, Switzerland). Cells are kept at 2 x10⁷ cells/ml (90% FCS, 10% DMSO) in cryotubes (Nunc) in liquid nitrogen until use.

The cells are incubated for four days at 37°C in a humidified CO₂ (7%) incubator in costar flasks at the concentration of 7 x 10⁵ cells/ml in culture medium containing RPMI 1640 (Gibco, Pacely, England) supplemented with Na-pyruvate (1 mM; Gibco), MEM nonessential amino acids and vitamins (Gibco), 2-mercaptoethanol (50 µM), L-glutamine (2 mM), gentamicin and penicillin/streptomycin (100 µg/ml; Gibco), bacto asparagine (20 □g/ml; Difco), human insulin (5 □g/ml; Sigma), human transferrin (40 □g/ml; Sigma), selected fetal calf serum (10%, Hyclone Laboratories, Logan, UT) and 100 µg/ml phytohemagglutinine. Cells are washed twice in RPMI 1640 medium containing 10% FCS and incubated for 2 hours. After centrifugation the cells are taken up in the culture medium mentioned above (without phytohemagglutinine) containing interleukin-2 (Chiron 200 U/ml), distributed in triplicates into flat-bottomed 96-well tissue culture plates (Costar #3596) at a concentration of 5 x 10⁴ cells/0.2 ml in the presence of appropriate concentrations of test compounds and incubated at 37°C for 72 hours. ³H-thymidine (1 □Ci/0.2 ml) was added for the last 16 hours of culture. Subsequently cells are harvested and counted on a scintillation counter.

Suitable JAK3 kinase inhibitors include e.g.
- Compounds as disclosed in USP 2003/0073719A1, e.g. a compound of formula XIV 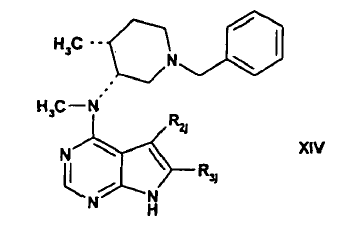 wherein
   each of R₂ⱼ and R₃ⱼ independently is selected from the group consisting of H, amino, halogen, OH, nitro, carboxy, C₂₋₆alkenyl, C₂₋₆alkynyl, CF₃, trifluoromethoxy, C₁₋₆alkyl, C₁₋₆ alkoxy, C₃₋₆cycloalkyl wherein the alkyl, alkoxy or cycloalkyl groups are optionally substituted by one to three groups selected from halogen, OH, carboxy, amino, C₁₋₆alkylthio, C₁₋₆ alkylamino, (C₁₋₆ alkyl)₂amino, C₅₋₉heteroaryl, C₂₋₉heterocycloalkyl, C₃₋₉cycloalkyl or C₆₋₁₀aryl; or each of R₂ⱼ and R₃ⱼ independently is C₃₋₁₀cycloalkyl, C₃₋₁₀cycloalkoxy, C₁₋₆alkylamino, (C₁₋₆ alkyl)₂amino, C₆₋₁₀arylamino, C₁₋₆alkylthio, C₆₋₁₀arylthio, C₁₋₆alkylsulfinyl, C₆₋₁₀arylsulfinyl, C₁₋₆alkylsulfonyl, C₆₋₁₀arylsulfonyl, C₁₋₆acyl, C₁₋₆alkoxy-CO-NH-, C₁₋₆alkylamino-CO-, C₅₋₉heteroaryl, C₂₋₉ heterocycloalkyl or C₆₋₁₀aryl wherein the heteroaryl, heterocycloalkyl and aryl groups are optionally substituted by one to three halogeno, C₁₋₆alkyl, C₁₋₆alkyl-CO-NH-, C₁₋₆alkoxy-CO-NH-, C₁₋₆alkyl-CO-NH-C₁₋₆alkyl, C₁₋₆alkoxy-CO-NH-C₁₋₆alkyl, C₁₋₆alkoxy-CO-NH-C₁₋₆ alkoxy, carboxy, carboxy-C₁₋₆alkyl, carboxy-C₁₋₆alkoxy, benzyloxycarbonyl-C₁₋₆alkoxy, C₁₋₆ alkoxycarbonyl-C₁₋₆alkoxy, C₆₋₁₀aryl, amino, aminoC₁₋₆alkyl, C₂₋₇alkoxycarbonylamino, C₆₋₁₀ aryl-C₂₋₇alkoxycarbonylamino, C₁₋₆alkylamino, (C₁₋₆alkylhamino, C₁₋₆alkylamino-C₁₋₆alkyl, (C₁₋₆alkyl)₂amino-C₁₋₆alkyl, hydroxy, C₁₋₆alkoxy, carboxy, carboxy-C₁₋₆alkyl, C₂₋₇alkoxycarbonyl, C₂₋₇alkoxycarbonyl-C₁₋₆alkyl, C₁₋₆alkoxy-CO-NH-, C₁₋₆alkyl-CO-NH-, cyano, C₅₋₉ heterocycloalkyl, amino-CO-NH-, C₁₋₆alkylamino-CO-NH-, (C₁₋₆alkylhamino-CO-NH-, C₆₋₁₀ arylamino-CO-NH-, C₅₋₉heteroarylamino-CO-NH-, C₁₋₆alkylamino-CO-NH-C₁₋₆alkyl, (C₁₋₆ alkyl)₂amino-CO-NH-C₁₋₆alkyl, C₆₋₁₀arylamino-CO-NH-C₁₋₆alkyl, C₅₋₉heteroarylamino-CO-NH-C₁₋₆alkyl, C₁₋₆alkylsulfonyl, C₁₋₆alkylsulfonylamino, C₁₋₆alkylsulfonylaminoC₁₋₆alkyl, C₆₋₁₀ arylsulfonyl, C₆₋₁₀arylsulfonylamino, C₆₋₁₀arylsulfonylamino-C₁₋₆alkyl, C₁₋₆alkylsulfonylamino, C₁₋₆alkylsulfonylamino-C₁₋₆alkyl, C₅₋₉heteroaryl or C₂₋₉heterocycloalkyl; for example methyl-[(3R,4R)-4-methyl-1-(propene-1-sulfonyl)-piperidin-3-yl]-(7H-pyrrolo[2,3-d]pyrimidin-4-yl)-amine; (3R,4R)-)-4-methyl-3-[methyl-(7H-pyrrolo[2- ,3-d]pyrimidin-4-yl)-amino]-piperidine-1-carboxylic acid methyl ester 3,3,3-trifluoro-1-{(3R,4R)-4-methyl-3-[methyl-(7H-pyrrolo[2,3-d]pyrimidin- -4-yl)-amino]-piperidin-1-yl}-propan-1-one; (3R,4R)-4-methyl-3-[methyl-(7H-pyrrolo[2,3-d]pyrimidin-4-yl)-amino]-piperidine-1-carboxylic acid dimethylamide; {(3R,4R)-4-methyl-3-[methyl-(7H-pyrrolo[2,3-d]pyrimidin-4-yl)-amino]-piperidine-1-carbonyl}-amino)-acetic acid ethyl ester; 3-{(3R,4R)-4-Methyl-3-[methyl-(7H-pyrrolo[2,3-d]pyrimidin-4-yl)-amino]-piperidin-1-yl}-3-oxo-propionitrile; 3,3,3-trifluoro-1-{(3R,4R)-4-methyl-3-[- methyl-(5-methyl-7H-pyrrolo[2,3-d]pyrimidin-4-yl)-amino]-piperidin-1-yl}-propan-1-one; 1-{(3R,4R)-4-methyl-3-[methyl-(7H-pyrrolo[2,3-d]pyrimidin-4-y-1)-amino]piperidin-1-yl}-but-3-yn-1-one; 1-{(3R,4R)-3-[(5-chloro-7H-pyrrol-o[2,3-d]pyrimidin-4-yl)-methyl-amino]-4-methyl-piperidin-1-yl}-propan-1-one; 1-{(3R,4R)-3-[(5-fluoro-7H-pyrrolo[2,3-d]pyrimidin-4-yl)-methyl-amino]-- 4-methyl-piperidin-1-yl}-propan-1-one; (3R,4R)-N-cyano-4-methyl-3-[methyl-- (7H-pyrrolo[2,3-d]pyrimidin-4-yl)-amino]-N'-propyl-piperidine-1-carboxamidine; or (3R,4R)-N-cyano-4,N',N'-trimethyl-3-[methyl-(7H-pyrrolo[2,3-d]pyrimidin-4-yl)-amino]-piperidine-1-carboxamidine;
- Compounds as disclosed in WO 02/092571, e.g. a compound of formula XV 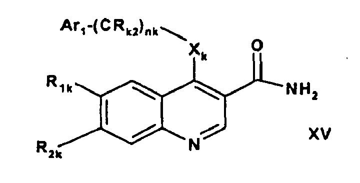 wherein
   Ar₁ is selected from phenyl, tetrahydronaphthenyl, indolyl, pyrazolyl, dihydroindenyl, 1-oxo-2,3-dihydroindenyl or indazolyl, each of which can be optionally substituted by one or more groups selected from halogen, hydroxy, cyano, C₁₋₈alkoxy, CO₂R₈ₖ, CONR₉ₖR₁₀ₖ, C₁₋₈alkyl-O-C₁₋₈alkyl, C₁₋₈alkyl-NR₈ₖ-C₁-₈alkyl, C₁₋₈alkyl-CONR₈-C₁₋₈alkyl, C₁₋₈alkyl-CONR₉ₖR₁₀ₖ, NR₈ₖCOC₁₋₈alkyl, C₁₋₈thioalkyl, C₁₋₈alkyl (itself optionally substituted by one or more OH or cyano or fluorine) or C₁₋₈alkoxy;
   Xₖ is NR₃ₖ or O; nₖ is 0 or 1;
   each Rₖ group independently is hydrogen or C₁₋₈alkyl;
   each of R₁ₖ and R₂ₖ independently is selected from H, halogen, nitro, cyano, C₁₋₈alkyl, C₁₋₈alkoxy, OH, aryl, Yₖ(CR₁₁ₖ₂)ₚₖNR₄ₖR₅ₖ, Yk(CR₁₁ₖ₂)ₚₖCONR₄ₖR₅ₖYₖ(CR₁₁ₖ₂)PkCO₂R₆ₖ, Yₖ(CR₁₁ₖ₂)pₖOR₆ₖ; Yₖ(C R₁₁ₖ₂)ₚₖ R₆ₖ; or R₁ₖ and R₂ₖ are linked together as -OCHO- or-OCH₂CH₂O-;
   each R₁₁ₖ independently is H,C₁₋₈alkyl, hydroxy or halogen; pₖ is 0, 1,2,3,4 or 5;
   R₃ₖ is H or C₁₋₈alkyl;
   Yₖ is oxygen, CH₂ or NR₇ₖR₃ₖ is hydrogen or C₁₋₈alkyl ;
   each of R₄ₖ and R₅ₖ independently is H, C₁₋₈alkyl or R₄ₖ and R₅ₖ together with the nitrogen atom to which they are attached form a 4-to 7-membered saturated or aromatic heterocyclic ring system optionally containing a further O, S or NR₆ₖ, or one of R₄ₖ and R₅ₖ is H or C₁₋₈ alkyl and the other is a 5-or 6-membered heterocyclic ring system optionally containing a further O, S or N atom;
   R₆ₖ is H, C₁₋₈alkyl, phenyl or benzyl;
   R₇ₖ is H orC₁₋₈alkyl ;
   R₈ₖ is H or C₁₋₈alkyl; each of R₉ₖ and R₁₀ independently is hydrogen or C₁₋₈alkyl;
- Compounds as disclosed in US 2002/0055514A1, e.g. a compound of formula XVI 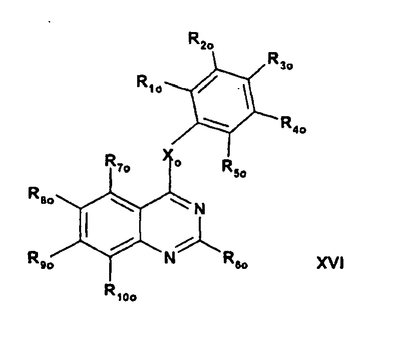 wherein
   Xₒ is NH, NR₁₁₀, S, O CH₂ or R₁₁₀CH;
   R₁₁₀ is H, C₁₋₄alkyl or C₁₋₄alkanoyl;
   each of R₁₀ to R₈₀, independently, is H, halogen, OH, mercapto, amino, nitro, C₁₋₄alkyl, C₁₋₄alkoxy or C₁₋₄alkylthio; wherein 2 of R₁₀-R₅₀ together with the phenyl ring to which they are attached may optionally form a fused ring, for example, forming a naphthyl or a tetrahydronaphthyl ring; and further wherein the ring formed by the two adjacent groups of R₁₀-R₅₀ may optionally be substituted by 1, 2, 3 or 4 halogen, hydroxy, mercapto, amino, nitro, C₁₋₄alkyl, C₁₋₄alkoxy or C₁₋₄alkylthio; and provided that at least one of R₂₀-R₅₀ is OH, and
   each of R₉₀ and R₁₀₀ independently is H, halogen, C₁-₄alkyl, C₁₋₄alkoxy or C₁₋₄alkanoyl; or R₉₀ and R₁₀₀ together are methylenedioxy;
- Compounds as disclosed in WO 04/052359, e.g. a compound of formula XVII 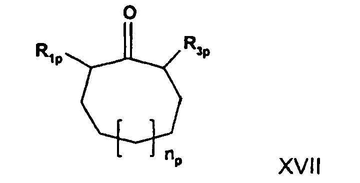
   wherein nₚ is 1,2,3,4 or 5;
   R₁ₚ is H, CH₃ or CH₂N(CH₃)₂; and
   R₃ₚ is CH₂N(CH₃)₂
   In free form or in a pharmaceutically acceptable salt form.

The compounds of formulae XIV to XVII may exist in free or salt form. Examples of pharmaceutically acceptable salts of the compounds of the formulae XIV to XVI include salts with inorganic acids, such as hydrochloride, salts with organic acids, such as acetate or citric acid, or, when appropriate, salts with metals such as sodium or potassium, salts with amines, such as triethylamine and salts with dibasic amino acids, such as lysine.

When the compounds of formulae XIV to XVII have one or more asymmetric centers in the molecule, the present invention is to be understood as embracing the various optical isomers, as well as racemates, diastereoisomers and mixtures thereof are embraced. When the compounds of formulae XIV to XVII comprise a double bond, the compounds may exist as cis or trans configurations or as mixtures thereof.

In formula XIV C₆₋₁₀aryl is phenyl or naphthyl. C₂₋₉heterocycloalkyl may be e.g. pyrrolidinyl, tetrahydrofuranyl, dihydrofuranyl, tetrahydropyranyl, pyranyl, thiopyranyl, aziridinyl, oxiranyl, methylenedioxy, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, piperidinyl, morpholinyl, piperazinyl, etc. Such a group will be attached either by a C or N atom. C₂₋₉heteroaryl may be e.g. furyl, thienyl, thiazolyl, pyrazolyl, isothizolyl, oxazolyl, isoxazolyl, pyrrolyl, triazolyl, tetrazolyl, imidazolyl, oxadiazolyl, thiadiazolyl, pyridyl, pyrimidyl, pyrazinyl, pyridazinyl, triazinyl, pyrazolo[3,4-b]pyridinyl, cinnolinyl, pteridinyl, purinyl, benzoxazolyl, benzothiazolyl, benzofuranyl, isoindolyl, indolyl, indolizinyl, indazolyl, isoquinolyl, quinolyl, phthalazinyl, quinoxalinyl, quinazolinyl, benzoxazinyl, etc.. Such a group will be attached either by a C or N atom.

Preferred JAK3 kinase inhibitors include e.g. N-benzyl-3,4-dihydroxy-benzylidene-cyanoacetamide α-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131),[4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, and 3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, e.g. mono-citrate (also called CP-690,550) or a compound of formula XVII.

In each case where citations of patent applications are given above, the subject matter relating to the compounds is hereby incorporated into the present application by reference. Comprised are likewise the pharmaceutically acceptable salts thereof, the corresponding racemates, diastereoisomers, enantiomers, tautomers as well as the corresponding crystal modifications of above disclosed compounds where present, e.g. solvates, hydrates and polymorphs, which are disclosed therein. The compounds used as active ingredients in the combinations of the invention can be prepared and administered as described in the cited documents, respectively. Also within the scope of this invention is the combination of more than two separate active ingredients as set forth above, i.e. a pharmaceutical combination within the scope of this invention could include three active ingredients or more.

In accordance with the particular findings of the present invention, there is provided
1. A pharmaceutical combination comprising:
   a) at least one S1P receptor agonist or modulator, and
   b) at least one JAK3 kinase inhibitor.
2. A method for treating or preventing an autoimmune disease or disorder, or cell, tissue or organ graft rejection in a subject in need thereof, comprising co-administration to said subject, e.g. concomitantly or in sequence, of a therapeutically effective amount of at least one S1 P receptor agonist or modulator, and at least one JAK3 kinase inhibitor, e.g. as disclosed above.

Examples of autoimmune diseases include e.g. sarcoidosis, fibroid lung, idiopathic interstitial pneumonia, obstructive airways disease, including conditions such as asthma, intrinsic asthma, extrinsic asthma, dust asthma, particularly chronic or inveterate asthma (for example late asthma and airway hyperreponsiveness), bronchitis, including bronchial asthma, infantile asthma, allergic rheumatoid arthritis, systemic lupus erythematosus, nephrotic syndrome lupus, Hashimoto's thyroiditis, multiple sclerosis, myasthenia gravis, type I diabetes mellitus and complications associated therewith, type II adult onset diabetes mellitus, uveitis, nephrotic syndrome, steroid dependent and steroid-resistant nephrosis, palmoplantar pustulosis, allergic encephalomyelitis, glomerulonephritis, psoriasis, psoriatic arthritis, atopic eczema (atopic dermatitis), contact dermatitis and further eczematous dermatitises, seborrheic dermatitis, lichen planus, pemphigus, bullous pemphigoid, epidermolysis bullosa, urticaria, angioedemas, vasculitides, erythemas, cutaneous eosinophilias, acne, alopecia areata, eosinophilic fasciitis, atherosclerosis, conjunctivitis, keratoconjunctivitis, keratitis, vernal conjunctivitis, uveitis associated with Behcet's disease, herpetic keratitis, conical cornea, dystorphia epithelialis comeae, keratoleukoma, ocular pemphigus, Mooren's ulcer, scleritis, Graves' ophthalmopathy, severe intraocular inflammation, inflammation of mucosa or blood vessels such as leukotriene B4-mediated diseases, gastric ulcers, vascular damage caused by ischemic diseases and thrombosis, ischemic bowel disease, inflammatory bowel disease (e.g. Crohn's disease and ulcerative colitis), necrotizing enterocolitis, renal diseases including interstitial nephritis, Goodpasture's syndrome hemolytic uremic syndrome and diabetic nephropathy, nervous diseases selected from multiple myositis, Guillain-Barre syndrome, Meniere's disease and radiculopathy, collagen disease including scleroderma, Wegener's granuloma and Sjogren' syndrome, chronic autoimmune liver diseases including autoimmune hepatitis, primary biliary cirrhosis and sclerosing cholangitis), partial liver resection, acute liver necrosis (e.g. necrosis caused by toxins, viral hepatitis, shock or anoxia), B-virus hepatitis, non-A/non-B hepatitis and cirrhosis, fulminant hepatitis, pustular psoriasis, Behcet's disease, active chronic hepatitis, Evans syndrome, pollinosis, idiopathic hypoparathyroidism, Addison disease, autoimmune atrophic gastritis, lupoid hepatitis, tubulointerstitial nephritis, membranous nephritis, amyotrophic lateral sclerosis or rheumatic fever.

By graft rejection is meant acute or chronic rejection of cells, tissue or solid organ allo- or xenografts of e.g. pancreatic islets, stem cells, bone marrow, skin, muscle, corneal tissue, neuronal tissue, heart, lung, combined heart-lung, kidney, liver, bowel, pancreas, trachea or oesophagus, or graft-versus-host diseases. Chronic rejection may also be named graft vessel diseases or graft vasculopathies.
3. A pharmaceutical combination as defined under 1) above, e.g. in the form of a kit which may further comprise instructions for the administration, e.g. for use in a method as defined under 2) above.
4. A pharmaceutical combination as defined under 1) above for use in the preparation of a medicament for use in a method as defined under 2) above.

Utility of the combination of the invention in a method as hereinabove specified, may be demonstrated in animal test methods as well as in clinic, for example in accordance with the methods hereinafter described.

### A1. Rat Heart transplantation

The strain combination used: Male Lewis (RT¹ haplotype) and BN (RT¹ haplotype). The animals are anaesthetised using inhalational isofluorane. Following heparinisation of the donor rat through the abdominal inferior vena cava with simultaneous exsanguination via the aorta, the chest is opened and the heart rapidly cooled. The aorta is ligated and divided distal to the first branch and the brachiocephalic trunk is divided at the first bifurcation. The left pulmonary artery is ligated and divided and the right side divided but left open. All other vessels are dissected free, ligated and divided and the donor heart is removed into iced saline.

The recipient is prepared by dissection and cross-clamping of the infra-renal abdominal aorta and vena cava. The graft is implanted with end-to-side anastomoses, using 10/0 monofilament suture, between the donor brachiocephalic trunk and the recipient aorta and the donor right pulmonary artery to the recipient vena cava. The clamps are removed, the graft tethered retroabdominally, the abdominal contents washed with warm saline and the animal is closed and allowed to recover under a heating lamp. Graft survival is monitored by daily palpation of the beating donor heart through the abdominal wall. Rejection is considered to be complete when heart beat stops. Increases of graft survival are obtained in animals treated with a combination acording to the invention, e.g. a combination of Compound A in the hydrochloride salt form and the compound CP-690,550 in the mono-citrate salt form, each component of the combination being administered orally at a daily dose of 0.1 to 50 mg/kg. Thus Compound A in the hydrochloride form, when administered at a dose of 0.3 to 3 mg/kg/day, and CP-690,550 mono-citrate, when administered at an EC₅₀ (drug concentration in blood at which 50% of the animals maintain their graft for 28 days) of 60 ng/ml, significantly increase the graft survival.

### A2. Islet Graft

Islets from BALB/C (H-2^{d}) mice are transplanted beneath the renal capsule of STZ-induced diabetic CBA (H-2^{k}) mice. The recipients are treated orally with a combination according to the invention for 50 days after islet transplantation, each component being preferably administered at a daily dose of 0.1 to 40 mg/kg. Functional status of the islet graft is monitored by measuring blood glucose daily. Normal glycemia is maintained for 80 ± 5 and >115 days in the treated animals compared with 10 ± 2 days in untreated animals, e.g. when animals are treated with 1 or 3 mg/kg/d of Compound A hydrochloride and 0.5 to 75 mg/kg/d CP-690,550 mono-citrate.

### B. Combined Treatment

Suitable clinical studies are, for example, open label, dose escalation studies in patients with psoriasis or multiple sclerosis. Such studies prove in particular the synergism of the active ingredients of the combination of the invention. The beneficial effects on psoriasis or multiple sclerosis can be determined directly through the results of these studies which are known as such to a person skilled in the art. Such studies are, in particular, suitable to compare the effects of a monotherapy using the active ingredients and a combination of the invention. Preferably, the dose of agent (a) is escalated until the Maximum Tolerated Dosage is reached, and agent (b) is administered with a fixed dose. Alternatively, the agent (a) is administered in a fixed dose and the dose of agent (b) is escalated. Each patient receives doses of the agent (a) either daily or intermittent. The efficacy of the treatment can be determined in such studies,

e.g., after 12, 18 or 24 weeks by evaluation of symptom scores every 6 weeks.

Alternatively, a placebo-controlled, double blind study can be used in order to prove the benefits of the combination of the invention mentioned herein, e.g. in transplantation of an organ, tissue or cells, e.g. Langerhans islet cells.

The administration of a pharmaceutical combination of the invention results not only in a beneficial effect, e.g. a synergistic therapeutic effect, e.g. with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically active ingredients used in the combination of the invention.

A further benefit is that lower doses of the active ingredients of the combination of the invention can be used, for example, that the dosages need not only often be smaller but are also applied less frequently, which may diminish the incidence or severity of side-effects. This is in accordance with the desires and requirements of the patients to be treated.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

It is one objective of this invention to provide a pharmaceutical composition comprising a quantity, which is jointly therapeutically effective against graft rejection or autoimmune diseases or disorders associated therewith comprising a combination of the invention. In this composition, agent a) and agent (b) may be administered together, one after the other or separately in one combined unit dosage form or in two separate unit dosage forms. The unit dosage form may also be a fixed combination.

The pharmaceutical compositions for separate administration of agent a) and agent b) or for the administration in a fixed combination, i.e. a single galenical composition comprising at least two combination partners a) and b), according to the invention may be prepared in a manner known per se and are those suitable for enteral, such as oral or rectal, and parenteral administration to mammals (warm-blooded animals), including humans, comprising a therapeutically effective amount of at least one pharmacologically active combination partner alone, e.g. as indicated above, or in combination with one or more pharmaceutically acceptable carriers or diluents, especially suitable for enteral or parenteral application.

Suitable pharmaceutical compositions contain, for example, from about 0.1 % to about 99.9%, preferably from about 1 % to about 60 %, of the active ingredient(s). Pharmaceutical preparations for the combination therapy for enteral or parenteral administration are, for example, those in unit dosage forms, such as sugar-coated tablets, tablets, capsules or suppositories, or ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the combination of the invention may be administered simultaneously or sequentially and in any order, and the components may be administered separately or as a fixed combination. For example, the method of preventing or treating graft rejection or autoimmune diseases according to the invention may comprise (i) administration of the first agent a) in free or pharmaceutically acceptable salt form and (ii) administration of an agent b) in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual combination partners of the combination of the invention may be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimens of simultaneous or alternating treatment and the term "administering" is to be interpreted accordingly.

The effective dosage of each of the combination partners employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A physician, clinician or veterinarian of ordinary skill can readily determine and prescribe the effective amount of the single active ingredients required to alleviate, counter or arrest the progress of the condition. Optimal precision in achieving concentration of the active ingredients within the range that yields efficacy without toxicity requires a regimen based on the kinetics of the active ingredients' availability to target sites.

Daily dosages for agent a) or b) or will, of course, vary depending on a variety of factors, for example the compound chosen, the particular condition to be treated and the desired effect. In general, however, satisfactory results are achieved on administration of agent a) at daily dosage rates of the order of ca. 0.03 to 5 mg/kg per day, particularly 0.1 to 5 mg/kg per day, e.g. 0.1 to 2.5 mg/kg per day, as a single dose or in divided doses. The S1P receptor agonist or modulator, e.g. a compound of formulae I to XIII, e.g. Compound A or B, may be administered by any conventional route, in particular enterally, e.g. orally, e.g. in the form of tablets, capsules, drink solutions or parenterally, e.g. in the form of injectable solutions or suspensions. Suitable unit dosage forms for oral administration comprise from ca. 0.02 to 50 mg active ingredient, usually 0.1 to 30 mg, e.g. Compound A or B, together with one or more pharmaceutically acceptable diluents or carriers therefor.

Agent b), e.g. CP-690,550 or a compound of formula XVII, may be administered to a human in a daily dosage range of 0.5 to 1000 mg. Suitable unit dosage forms for oral administration comprise from ca. 0.1 to 500 mg active ingredient, together with one or more pharmaceutically acceptable diluents or carriers therefor.

The administration of a pharmaceutical combination of the invention results not only in a beneficial effect, e.g. a synergistic therapeutic effect, e.g. with regard to inhibiting graft rejection in transplanted patients or slowing down or arresting autoimmune disorders, but also in further surprising beneficial effects, e.g. less side-effects, an improved quality of life or a decreased morbidity, compared to a monotherapy applying only one of the pharmaceutically active ingredients used in the combination of the invention.

A further benefit is that lower doses of the active ingredients of the combination of the invention can be used, for example, that the dosages need not only often be smaller but are also applied less frequently, or can be used in order to diminish the incidence of side-effects. This is in accordance with the desires and requirements of the patients to be treated.

A preferred combination is the combination of FTY720 hydrochloride with CP-690,555 monocitrate.

## Claims

1. A pharmaceutical combination comprising:
a) at least one S1 P receptor agonist or modulator, and
b) at least one JAK3 kinase inhibitor,
wherein the S1 P receptor agonist or modulator a) is selected from 2-amino-2-[2-(4-octylphenyl) ethyl]propane-1,3-diol (FTY720), in free form or in a pharmaceutically acceptable salt form, FTY720-phosphate, 2-amino-2-[4-(3-benzyloxyphenoxy)-2-chlorophenyl]propyl-1,3-propane-diol and 2-amino-2-[4-(benzyloxyphenylthio)-2-chlorophenyl]propyl-1,3-propane-diol, in free form or in a pharmaceutically acceptable salt or hydrate form.

2. A pharmaceutical combination according to claim 1 wherein the S1 P receptor agonist or modulator a) is 2-amino-2-[2-(4-octylphenyl) ethyl]propane-1,3-diol (FTY720), in free form or in a pharmaceutically acceptable salt form.

3. A pharmaceutical combination according to claim 1 or 2 wherein agent b) is selected from
- a compound of formula XIV 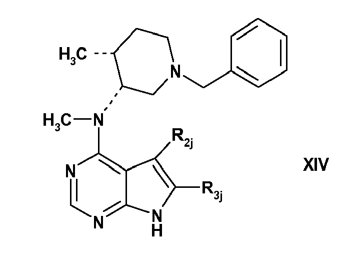 wherein
each of R₂ⱼ and R₃ⱼ independently is selected from the group consisting of H, amino, halogen, OH, nitro, carboxy, C₂₋₆alkenyl, C₂₋₆alkynyl, CF₃, trifluoromethoxy, C₁₋₆alkyl, C₁₋₆ alkoxy, C₃-₆cycloalkyl wherein the alkyl, alkoxy or cycloalkyl groups are optionally substituted by one to three groups selected from halogen, OH, carboxy, amino, C₁₋₆alkylthio, C₁₋₆ alkylamino, (C₁₋₆ alkyl)₂amino, C₅₋₉heteroaryl, C₂₋₉heterocycloalkyl, C₃₋₉cycloalkyl or C₆₋₁₀aryl; or each of R₂ⱼ and R₃ⱼ independently is C₃₋₁₀cycloalkyl, C₃₋₁₀cycloalkoxy, C₁₋₆alkylamino, (C₁₋₆alkyl)₂amino, C₆₋₁₀arylamino, C₁₋₆alkylthio, C₆₋₁₀arylthio, C₁₋₆alkylsulfinyl, C₆₋₁₀arylsulfinyl, C₁₋₆alkylsulfonyl, C₆₋₁₀arylsulfonyl, C₁₋₆acyl, C₁₋₆alkoxy-CO-NH-, C₁₋₆alkylamino-CO-, C₅₋₉heteroaryl, C₂₋₉ heterocycloalkyl or C₁₋₆aryl wherein the heteroaryl, heterocycloalkyl and aryl groups are optionally substituted by one to three halogeno, C₁₋₆alkyl, C₁₋₆alkyl-CO-NH-, C₁₋₆alkoxy-CO-NH-, C₁₋₆alkyl-CO-NH-C₁₋₆alkyl, C₁₋₆alkoxy-CO-NH-C₁₋₆alkyl, C₁₋₆alkoxy-CO-NH-C₁₋₆ alkoxy, carboxy, carboxy-C₁₋₆alkyl, carboxy-C₁₋₆alkoxy, benzyloxycarbonyl-C₁₋₆alkoxy, C₁₋₆ alkoxycarbonyl-C₁₋₆alkoxy, C₆₋₁₀aryl, amino, aminoC₁₋₆alkyl, C₂₋₇alkoxycarbonylamino, C₆₋₁₀ aryl-C₂₋₇alkoxycarbonylamino, C₁₋₆alkylamino, (C₁₋₆alkyl)₂amino, C₁₋₆alkylamino-C₁₋₆alkyl, (C₁₋₆alkyl)₂amino-C₁₋₆alkyl, hydroxy, C₁₋₆alkoxy, carboxy, carboxy-C₁₋₆alkyl, C₂₋₇alkoxycarbonyl, C₂₋₇alkoxycarbonyl-C₁₋₆alkyl, C₁₋₆alkoxy-CO-NH-, C₁₋₆alkyl-CO-NH-, cyano, C₅₋₉heterocycloalkyl, amino-CO-NH-, C₁₋₆alkylamino-CO-NH-, (C₁₋₆alkyl)₂amino-CO-NH-, C₆₋₁₀ arylamino-CO-NH-, C₅₋₉heteroarylamino-CO-NH-, C₁₋₆alkylamino-CO-NH-C₁₋₆alkyl, (C₁₋₆ alkyl)₂amino-CO-NH-C₁₋₆alkyl, C₁₋₆arylamino-CO-NH-C₁₋₆alkyl, C₅₋₉heteroarylamino-CO-NH-C₁₋₆alkyl, C₁₋₆alkylsulfonyl, C₁₋₆alkylsulfonylamino, C₁₋₆alkylsulfonylaminoC₁₋₆alkyl, C₆₋₁₀ arylsulfonyl, C₆₋₁₀arylsulfonylamino, C₆-₁₀arylsulfonylamino-C₁₋₆alkyl, C₁₋₆alkylsulfonylamino, C₁₋₆alkylsulfonylamino-C₁₋₆alkyl, C₅₋₉heteroaryl or C₂₋₉heterocycloalkyl;
- a compound of formula XV 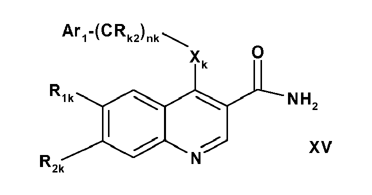 wherein
Ar₁ is selected from phenyl, tetrahydronaphthenyl, indolyl, pyrazolyl, dihydroindenyl, 1-oxo-2,3-dihydroindenyl or indazolyl, each of which can be optionally substituted by one or more groups selected from halogen, hydroxy, cyano, C₁₋₈alkoxy, CO₂R₈ₖ, CONR₉ₖR₁₀ₖ, C₁₋₈alkyl-O-C₁₋₈alkyl, C₁₋₈alkyl-NR₈ₖ-C₁₋₈alkyl, C₁₋₈alkyl-CONR₈-C₁₋₈alkyl, C₁₋₈alkyl-CONR₉ₖR₁₀ₖ, NR₈ₖCOC₁₋₈alkyl, C₁₋₈thioalkyl, C₁₋₈alkyl (itself optionally substituted by one or more OH or cyano or fluorine) or C₁₋₈alkoxy;
Xₖ is NR₃ₖ or O; nₖ is 0 or 1;
each Rₖ group independently is hydrogen or C₁₋₈alkyl ;
each of R₁ₖ and R₂ₖ independently is selected from H, halogen, nitro, cyano, C₁₋₈alkyl, C₁₋₈alkoxy, OH, aryl, Yₖ(CR₁₁ₖ₂)ₚₖNR₄ₖR₅ₖ, Yₖ(CR₁₁ₖ₂)ₚₖCONR₄ₖR₅ₖYₖ(CR₁₁ₖ₂)ₚₖCO₂R₆ₖ, Yₖ(CR₁₁ₖ₂)ₚₖOR₆ₖ Yₖ(C R₁₁ₖ₂)ₚₖ R₆ₖ; or R₁ₖ and R₂ₖ are linked together as -OCHO- or-OCH₂CH₂O- ;
each R₁₁ₖ independently is H,C₁₋₈alkyl, hydroxy or halogen; pₖ is 0, 1,2,3,4 or 5;
R₃ₖ is H or C₁₋₈alkyl;
Yₖ is oxygen, CH₂ or NR₇ₖR₃ₖ is hydrogen or C₁₋₈alkyl ;
each of R₄ₖ and R₅ₖ independently is H, C₁₋₈alkyl or R₄ₖ and R₅ₖ together with the nitrogen atom to which they are attached form a 4-to 7-membered saturated or aromatic heterocyclic ring system optionally containing a further O, S or NR₆ₖ, or one of R₄ₖ and R₅ₖ is H or C₁₋₈ alkyl and the other is a 5-or 6-membered heterocyclic ring system optionally containing a further O, S or N atom;
R₆ₖ is H, C₁₋₈alkyl, phenyl or benzyl;
R₇ₖ is H orC₁₋₈alkyl ;
R₈ₖ is H or C₁₋₈alkyl; each of R₉ₖ and R₁₀ independently is hydrogen or C₁₋₈alkyl;
- a compound of formula XVI 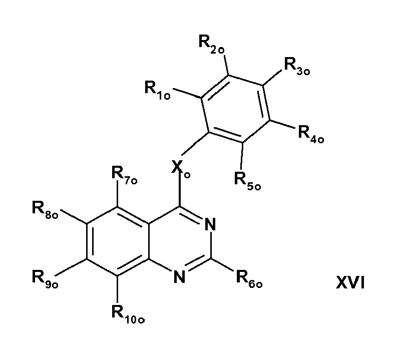 wherein
Xₒ is NH, NR₁₁₀, S, O CH₂ or R₁₁₀CH;
R₁₁₀ is H, C₁₋₄alkyl or C₁₋₄alkanoyl;
each of R₁₀ to R₈₀, independently, is H, halogen, OH, mercapto, amino, nitro, C₁₋₄alkyl, C₁₋₄alkoxy or C₁₋₄alkylthio; wherein 2 of R₁₀-R₅₀ together with the phenyl ring to which they are attached may optionally form a fused ring, for example, forming a naphthyl or a tetrahydronaphthyl ring; and further wherein the ring formed by the two adjacent groups of R₁₀-R₅₀ may optionally be substituted by 1, 2, 3 or 4 halogen, hydroxy, mercapto, amino, nitro, C₁₋₄alkyl, C₁₋₄alkoxy or C₁₋₄alkylthio; and provided that at least one of R₂₀-R₅₀ is OH, and each of R₉₀ and R₁₀₀ independently is H, halogen, C₁₋₄alkyl, C₁₋₄alkoxy or C₁₋₄alkanoyl; or R₉₀ and R₁₀₀ together are methylenedioxy; and
- a compound of formula XVII 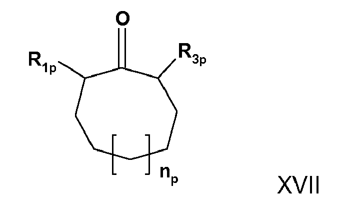
wherein nₚ is 1,2,3,4 or 5;
R₁p is H, CH₃ or CH₂N(CH₃)₂; and
R₃ₚ is CH₂N(CH₃)₂,
or a pharmaceutically acceptable salt thereof.

4. A pharmaceutical combination according to any one of claim 1 to 3, wherein the JAK3 kinase inhibitor b) is 3-{(3R,4R)-4-methyl-3-[methyl-(7H-pyrrolo[2,3-d]pyrimidin-4-yl)-amino]-piperidin-1-yl}-3-oxo-propionitrile.

5. A pharmaceutical combination according to any one of claim 1 to 3, wherein the JAK3 kinase inhibitor b) is a compound or formula XVII as defined in claim 3, in free form or in a pharmaceutically acceptable salt form.

6. A pharmaceutical combination according to any one of claim 1 to 3, for use in a method for treating or preventing an autoimmune disease or disorder, or cell, tissue or organ graft rejection.

7. A pharmaceutical combination according to any one of claim 1 to 3, in the form of a kit.

8. A pharmaceutical combination according to any one of claim 1 to 3, for use in the preparation of a kit for use in a method for treating or preventing an autoimmune disease or disorder, or cell, tissue or organ graft rejection.
